# EUROPEAN PATENT APPLICATION

(11) **EP 3 900 788 A1**
(43) Date of publication of application: **27.10.2021**
(21) Application number: 20382318.2
(22) Date of filing: 21.04.2020
(51) Int. Cl.: A61P 25/28, A61K 31/00, A61K 31/44

(54) **NON-PRODUCTIVE ANGIOGENESIS INHIBITOR FOR USE IN THE TREATMENT OF ALZHEIMER'S DISEASE**

(71) Applicant: Consejo Superior de Investigaciones Científicas (CSIC), 28006 Madrid (ES)
(72) Inventor: PASCUAL BRAVO, Alberto, 41013 Sevilla (ES); ROSALES NIEVES, Alicia Elena, 41013 Sevilla (ES); ÁLVAREZ VERGARA, María Isabel, 41013 Sevilla (ES)
(74) Representative: Hoffmann Eitle

(57) **Abstract**

Non-productive angiogenesis inhibitor for use in the treatment of Alzheimer's disease. The present invention refers to non-productive angiogenesis inhibitors for use in the treatment of Alzheimer's disease (AD). Moreover, the present invention also refers to a pharmaceutical composition comprising non-productive angiogenesis inhibitors and, optionally, pharmaceutically acceptable excipients or carriers, for use in the treatment of AD. Finally, the present invention also covers *in vitro* methods for the identification and production of candidate compounds for the treatment of AD or for monitoring the response to a treatment against AD.

## Description

### FIELD OF THE INVENTION

The present invention refers to the medial field. Precisely, the present invention refers to non-productive angiogenesis inhibitors for use in the treatment of Alzheimer's disease (AD). Moreover, the present invention also refers to a pharmaceutical composition comprising non-productive angiogenesis inhibitors and, optionally, pharmaceutically acceptable excipients or carriers, for use in the treatment of AD. Finally, the present invention also covers *in vitro* methods for the identification and/or production of candidate compounds for the treatment of AD or for monitoring the response to a treatment against AD.

### PRIOR ART

More than one century ago, Alois Alzheimer described a dementia case where he found "minute miliary foci which are caused by the deposition of a special substance in the cortex", a pathological finding nowadays termed senile plaque. These deposits are mainly composed of aggregated forms of the amyloid β (Aβ) peptide, which is generated by the sequential action of two proteases, the β-secretase/BACE1 and the γ-secretase complex -whose catalytic subunit is Presenilin (PSEN1/2)-, on the Aβ Precursor Protein (APP). Most of AD cases are sporadic late onset AD (LOAD), whereas only a small percentage of patients have familiar AD (fAD) with an earlier onset and a more aggressive clinical course. fAD associated mutations, either in *APP, PSEN1,* or *PSEN2* locus, correlated with either higher production of toxic Aβ₁₋₄₂ species (*APP*) or a higher ratio of Aβ₁₋₄₂/Aβ₁₋₄₀ (*PSEN*), but intriguingly, *PSEN* fAD variants are loss of function mutations with reduced processing of other substrates like Notch. Age is the main risk for developing LOAD and it has been shown that Notch processing by γ-secretase activity is decreased during aging in humans.

Notch signalling is involved in many different cellular processes, including the growing of new vessels by angiogenesis and the generation and maintenance of the blood-brain barrier (BBB), in coordination with the Wnt/β-catenin signalling pathway. Remarkably, in his first case report, Alzheimer also described changes in the vascular system remarking that "there is no infiltration of the vessels, however, a growth appears on the endothelia, in some places also a proliferation of vessels", suggesting an exacerbated underlying angiogenic process in AD. Puzzlingly, cerebral microvasculature is rather decreased specially around Aβ deposits in the human AD brain and in AD mice, although Aβ plaques accumulate angiogenic/hypoxic markers.

Angiogenesis is started by the inflammation/hypoxia-mediated induction of the vascular endothelial growth factor (VEGF), which binds to VEGFR2 in mature endothelial cells. Upon receptor-ligand binding, VEGF induces the extrusion of activated tip cells from the capillaries. These cells are characterized by the mobilization of the extracellular matrix, loss of the BBB, reduction of the blood-conducting lumen, and emission of filopodia that will guide the extension of the new vessels. VEGF-activated tip cells induce the conversion of contiguous cells to the stalk phenotype -characterized by the repression of VEGFR2 expression, proliferation, generation of vessel lumen, and activation of the BBB genetic program- through lateral inhibition. Molecularly, lateral inhibition is mediated by the activation of Delta-like 4 (Dll4) in tip cells that, in turn, signal over Notch transmembrane receptors expressed by the adjacent cells, in a process requiring the activity of the γ-secretase complex. Even partial inhibition of the Dll4/Notch pathway produces the initiation of pathologic angiogenesis that disassembles mature blood vessels into non-conducting tip cells, in a process termed non-productive angiogenesis (NPA).

Precisely, the inventors of the present invention have identified that NPA is playing an important role in the development of AD. Consequently, since there is an unmet medical need of finding effective treatments for AD, the present invention is focused on solving this problem by using a new approach for the treatment of AD which is based on the inhibition of NPA.

### DESCRIPTION OF THE INVENTION

### Brief description of the invention

As explained above, the present invention is directed to the treatment of AD, particularly by means of the inhibition of NPA.

The inventors of the present invention initially hypothesized that a failure of Notch-mediated lateral inhibition during angiogenesis could contribute to explain the accumulation of angiogenic markers and the reduction in blood vessels observed in the human AD brain. So, they first described that angiogenesis concentrates around Aβ deposits both in the human AD brain (Integrin-αvβ3 positive cells) and in an AD mouse model (VEGFA and Integrin-αvβ3 positive cells). Secondly, they identified the differentially expressed genes between AD and control mouse endothelial cells and showed that they have the molecular signature of NPA. Morphologically, two different AD mouse models accumulate abnormal vascular structures around Aβ plaques revealed with an NPA marker (IB4). In adults, genetic reduction of Notch-mediated lateral inhibition (genetic induction of NPA) in cerebral endothelial cells produces similar vascular scars (VaS) in the absence of Aβ overexpression. The VaS in the brain of AD mouse models replace blood vessels. Moreover, they showed that the blood vessel around Aβ plaques are abnormally surrounded by microglia and that vessel material is found in microglial phagocytic pouches. Correspondingly, inhibition of Notch mediated signalling induces vessel phagocytosis by microglia in the absence of Aβ deposition, indicating that NPA is by itself enough to stimulate vessel loss.

Interestingly, the inventors of the present invention have shown a reduction of Aβ load by blocking NPA, thus suggesting a better clearance of Aβ deposits by increased vascular function.

So, the first embodiment of the present invention refers to NPA inhibitors for use in the treatment of AD. Alternatively, this first embodiment refers to a method for treating AD which comprises the administration of a therapeutically effective dose or amount of an NPA inhibitor, or a pharmaceutical composition comprising NPA inhibitors and, optionally, pharmaceutically acceptable excipients or carriers. When the NPA is inhibited, the differentiation of endothelial cells into tip cells is halted and the abnormal vasculature is normalized, avoiding their phagocytosis by microglial cells.

### NPA inhibitors

Such as it is demonstrated in the results included in this patent application, the contribution that the inventors have made over the prior art is the inhibition of NPA as an innovative approach for the treatment of AD, irrespective of the NPA inhibitor which is finally used, and no matter if the inhibitor is a biological or a chemical molecule.

So, although the NPA inhibitor Sorafenib is used in the present invention as proof of concept, any NPA inhibitor could be used according to the present invention, for instance: Axitinib, Cabozantinib, Everolimus, Lenalidomide, Pazopanib, Regorafenib, Sunitinib, Thalidomide, Vandetanib, Ziv-aflibercept, Lapatinib, Lenvatinib, Ponatinib, Dasatinib, Nilotinib, Regorafenib, or any combination thereof.

So, the present invention opens the possibility of repositioning drugs, specifically angiogenesis inhibitors, for the treatment of a new indication such as AD.

### Pharmaceutical composition

The second embodiment of the present invention refers to a pharmaceutical composition comprising NPA inhibitors and, optionally, pharmaceutically acceptable excipients or carriers, for use in the treatment of Alzheimer's disease. In a preferred embodiment, the NPA inhibitor is selected from the group comprising: Sorafenib, Axitinib, Cabozantinib, Everolimus, Lenalidomide, Pazopanib, Regorafenib, Sunitinib, Thalidomide, Vandetanib, Ziv-aflibercept, Lapatinib, Lenvatinib, Ponatinib, Dasatinib, Nilotinib, Regorafenib or any combination thereof. In a preferred embodiment, the pharmaceutical composition comprises Sorafenib and, optionally, pharmaceutically acceptable excipients or carriers.

### Method for the identification and production of candidate compounds for the treatment of AD

The third embodiment of the present invention refers to an *in vitro* method for the identification and production of candidate compounds for the treatment of AD comprising: a) determining whether the NPA has been inhibited, or the abnormal vasculature has been normalized, after administering the candidate molecule, preferably by measuring the number of filopodia emitting cells and/or the number of filopodia per cell, as indicative of the transformation between endothelial and tip cells (thus, the presence or the number of tips cells is determined by measuring the number of filopodia emitting cells and/or the number of filopodia per cell), and b) where, if after administering the candidate molecule, the NPA is inhibited, or the abnormal vasculature has been normalized (because, for instance, the number of tip cells has been reduced), this is indicative that the candidate molecule is effective in the treatment of AD

In a preferred embodiment, the identification and production of candidate compounds for the treatment of AD comprises a) determining the level of expression or the presence/absence of IB4 and/or laminin after administering the candidate molecule, and b) wherein, if after administering the candidate molecule, the number of tip cells is reduced (measured by IB4 staining) and/or laminin expression is increased, this is indicative that the candidate molecule is effective in the treatment of AD

### Method for monitoring the response to a treatment against AD

The fourth embodiment of the present invention refers to an *in vitro* method for monitoring the response to a treatment against AD comprising: a) determining whether the NPA has been inhibited, or the abnormal vasculature has been normalized, after administering the candidate molecule, preferably by measuring the number of filopodia emitting cells and/or the number of filopodia per cell, as indicative of the transformation between endothelial and tip cells (thus, the presence or the number of tips cells is determined by measuring the number of filopodia emitting cells and/or the number of filopodia per cell), and b) where, if after administering the candidate molecule, the NPA is inhibited, or the abnormal vasculature has been normalized (because, for instance, the number of tip cells has been reduced), this is indicative that the treatment is effective against AD

In a preferred embodiment, the method for monitoring the response to a treatment against AD comprises: a) determining the level of expression or the presence/absence of IB4 and/or laminin after administering the candidate molecule, and b) wherein, if after administering the candidate molecule, the number of tip cells is reduced (measured by IB4 staining) and/or laminin expression is increased, this is indicative that the treatment is effective against AD

For the purpose of the present invention the following terms are defined:
- The term "non-productive angiogenesis (NPA)" refers to a pathologic angiogenesis wherein mature blood vessels are dissembled into non-conducting tip cells which are eliminated by phagocytosis. When the NPA is inhibited according to the present invention, the differentiation of endothelial cells into tip cells is halted, the phagocytosis is prevented, and the abnormal vasculature is normalized.
- The term "comprising" includes, but it is not limited to, whatever follows the word "comprising". Thus, use of the term "comprising" indicates that the listed elements are required or mandatory, but that other elements are optional and may or may not be present.
- By "consisting of' includes, and is limited to, whatever follows the phrase "consisting of'. Thus, the phrase "consisting of' indicates that the listed elements are required or mandatory, and that no other elements may be present.
- By "therapeutically effective dose or amount" is intended an amount that, when administered as described herein, brings about a positive therapeutic response in the patient. The exact amount required will vary from subject to subject, depending on (non-exhaustive list): the species, age, and general condition of the subject, the severity of the condition being treated or the mode of administration. An appropriate "effective" amount in any individual case may be determined by one of ordinary skill in the art using routine experimentation, based upon the information provided herein.

### Description of the figures

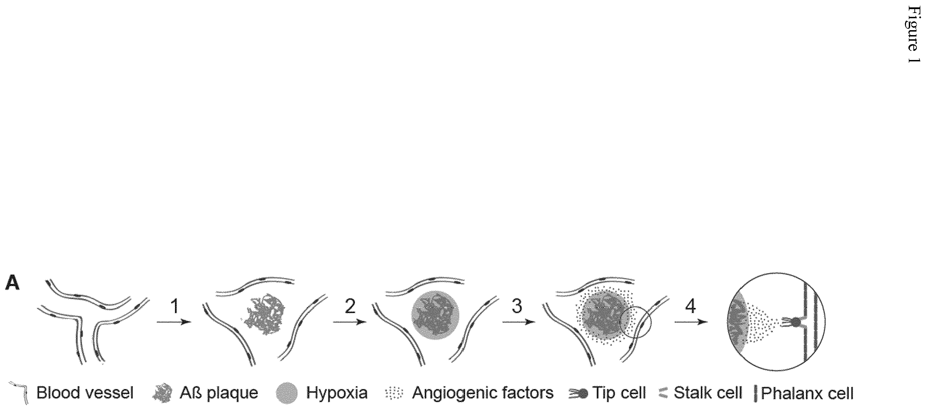

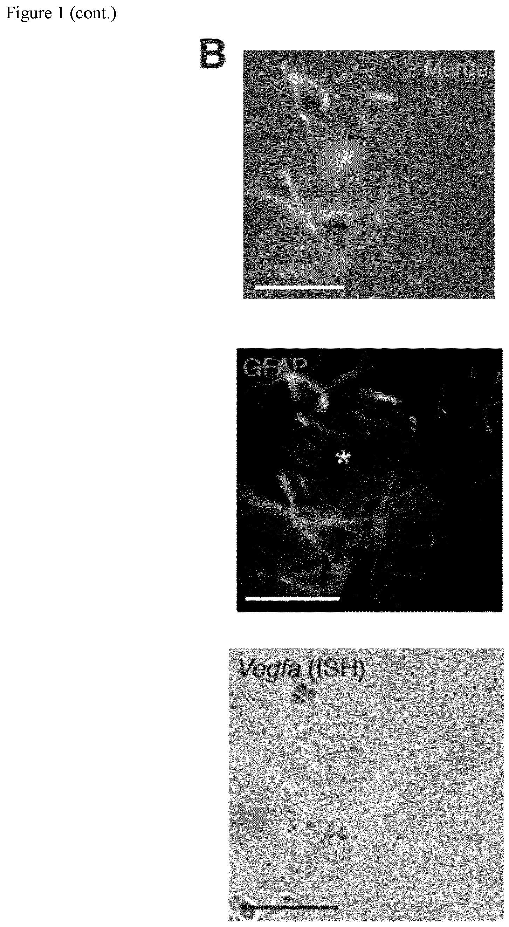

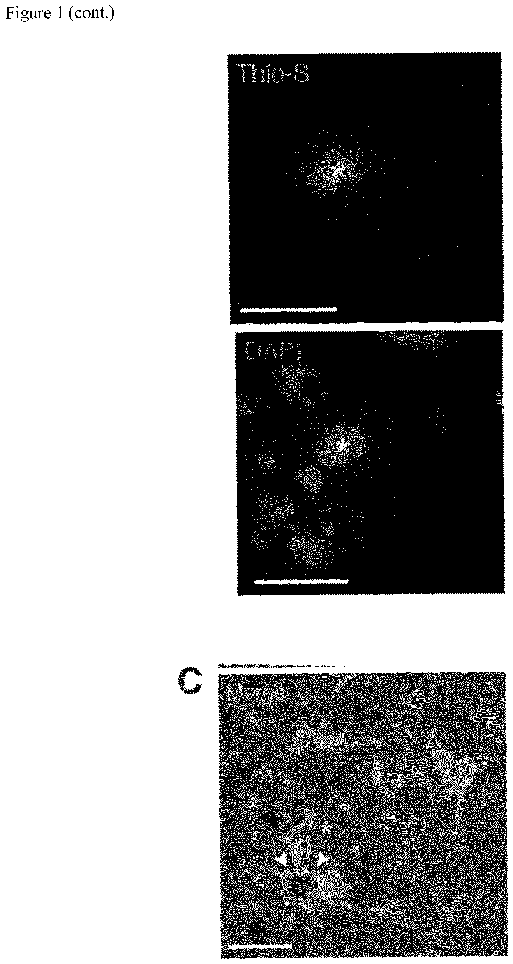

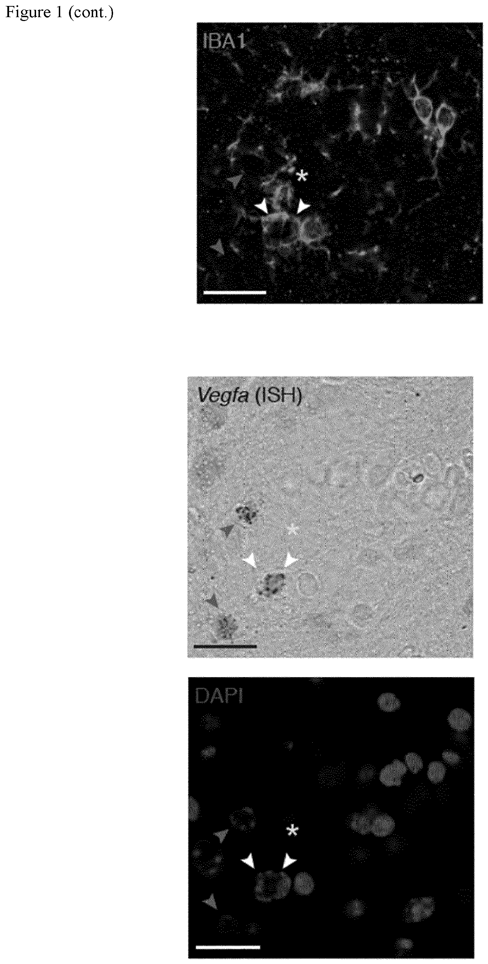

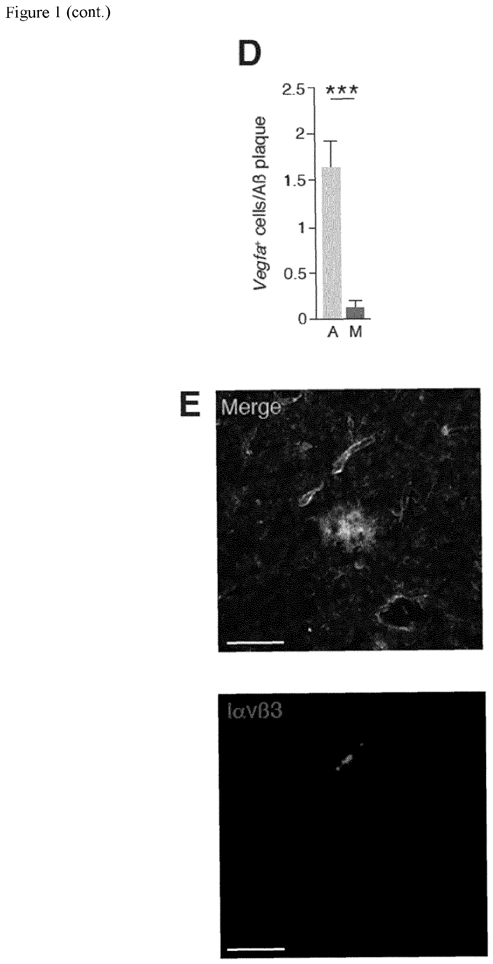

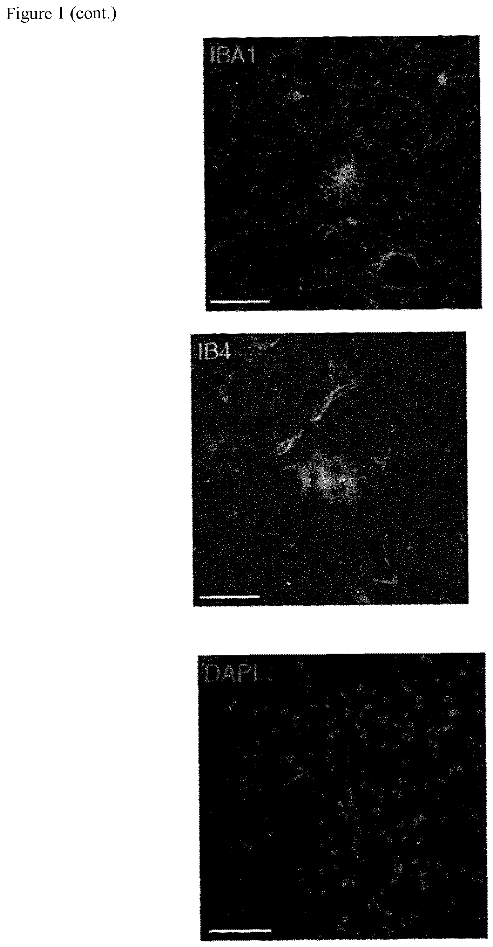

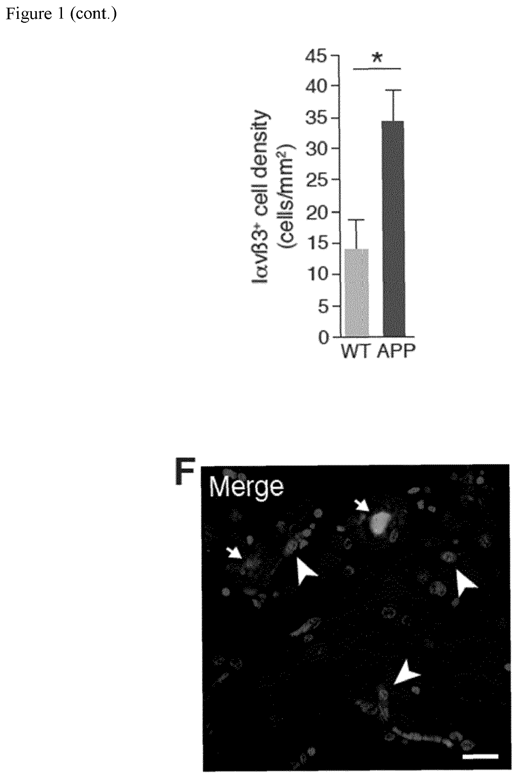

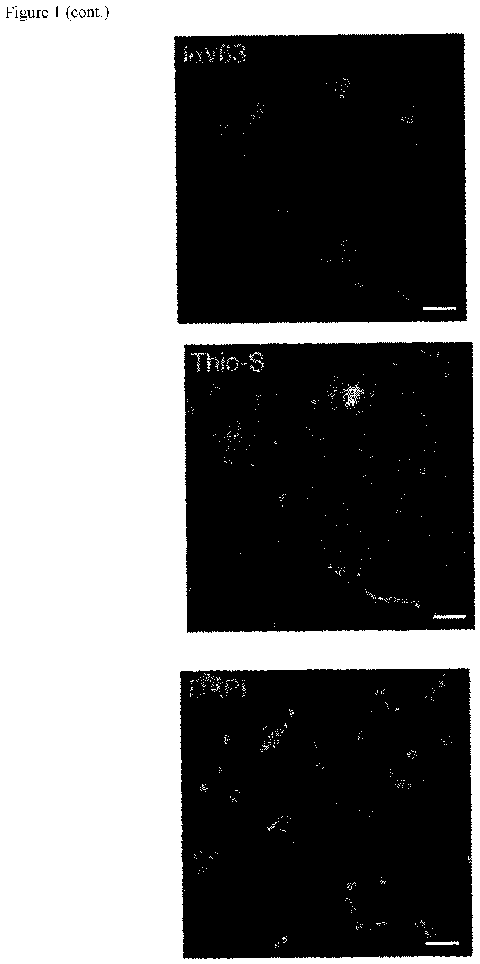

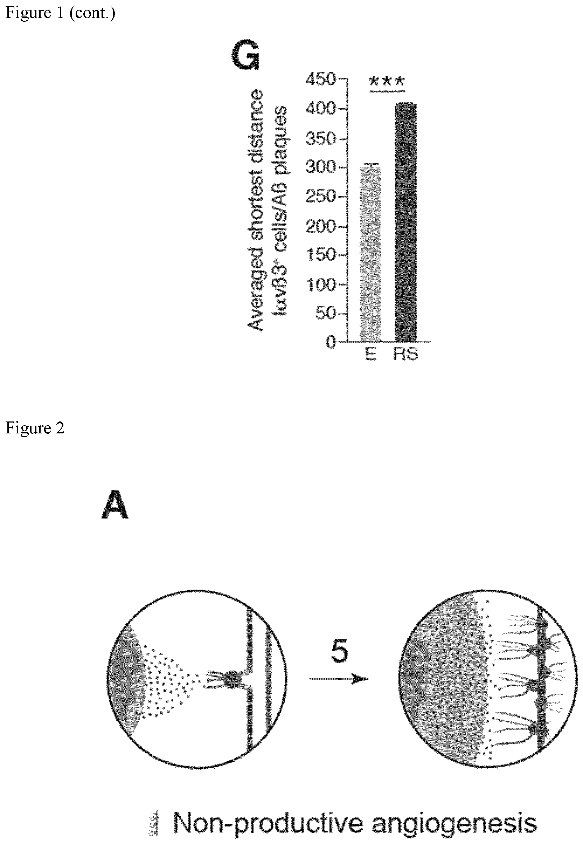

**Figure 1****. Angiogenesis is concentrated around Aβ plaques in AD. (A)** Working model of the angiogenesis around Aβ plaques. Aβ deposition separate vessels (1) producing local hypoxia (2) and inducing angiogenic factors expression (3). VEGF differentiates phalanx cells to tip cells that extrude from the vessel and stalk cells that will produce the new capillary (4). **(B-D)** *Vegfa* is expressed by astrocytes **(B)** and microglial cells **(C)** around Aβ plaques in 8-month-old *APP-PSEN1*/+ mice. Aβ plaques are autofluorescent in blue (yellow asterisks). Confocal XY images of coronal brain slices from and stained with astrocytic (GFAP; cyan; **B**), *Vegfa* (*in situ hybridization,* ISH; brown), Aβ (Thio-S; green; **B**), microglial (IBA1; green, **C**), and nuclear (DAPI; blue) markers. White arrowheads indicate microglial cells expressing *Vegfa* and red arrowheads point to a non-microglial *Vegfa*-expressing cell **(C).** Scale bars are 20 µm. **(D)** Quantification of the number of astrocytes (A) and microglia (M) expressing *Vegfa* mRNA per Aβ plaque. Mean ± SEM. *n* = 25 Aβ plaques from 5 mice; Student's *t*-test *** *p <* 0.001. **(E)** A cortical mouse brain slice stained with angiogenic endothelial (Integrin αvβ3 - Iαvβ3-; red), microglial (IBA1; green), endothelial (IB4; white), and nuclear (DAPI; blue) markers. Scale bars are 20 µm. Right bar graph, quantification of the Iαvβ3⁺ cell density in 8-month-old wild-type and *APP-PSEN1*/+ mice. Mean ± SEM. *n =* 5 mice per experimental group; Student's *t*-test * *p* < 0.05. **(F)** A human hippocampal brain slice stained with angiogenic endothelial (Iαvβ3; red), Aβ (Thio-S; green), and nuclear (DAPI; blue) markers. Scale bars are 20 µm. Arrowheads indicate angiogenic cells and arrows signal Aβ plaques. **(G)** Quantification of the shortest distance between Iαvβ3⁺ cells and Aβ plaques in four experimental (E) human brain slices and in ten random simulations (RS) of Iαvβ3⁺ cells location. Mean ± SEM. *n* = 2,995 (E) and 35,940 (RS) Iαvβ3⁺ cells and 573 Aβ plaques; Student's *t*-test *** *p* < 0.001.

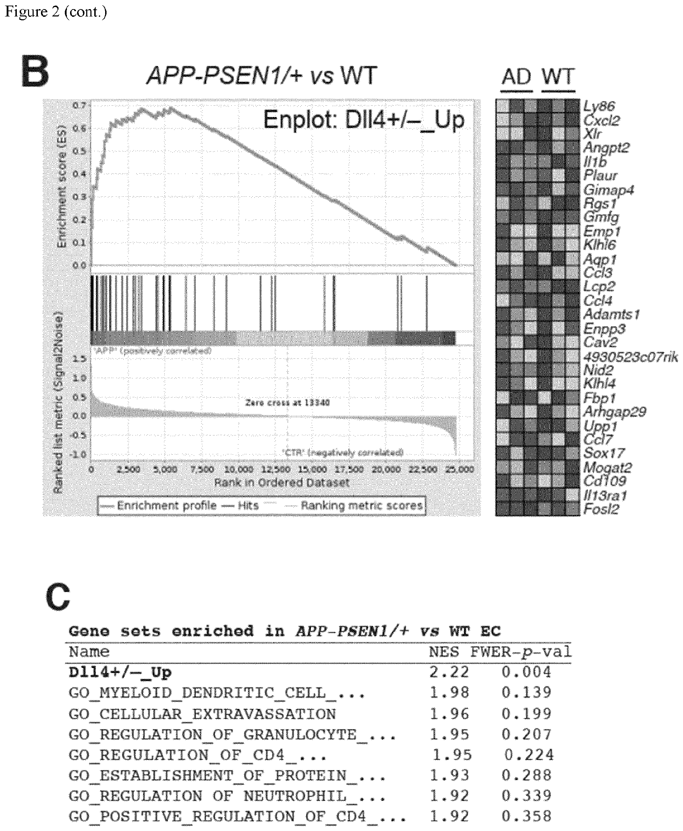

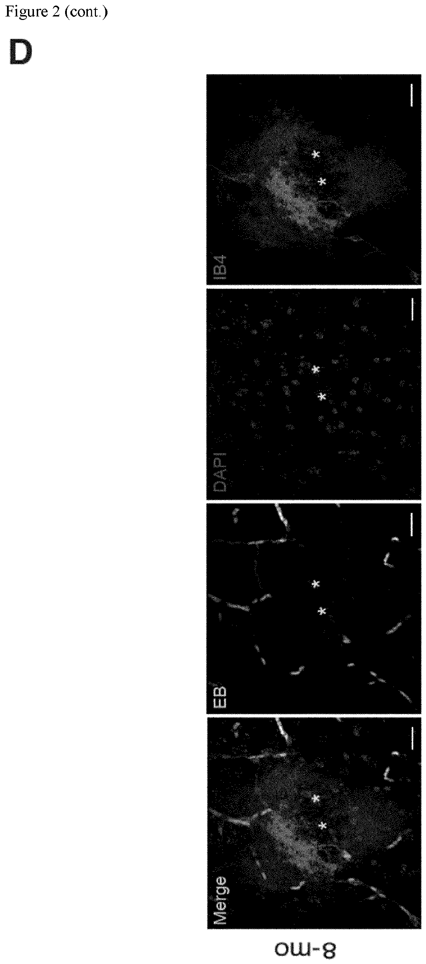

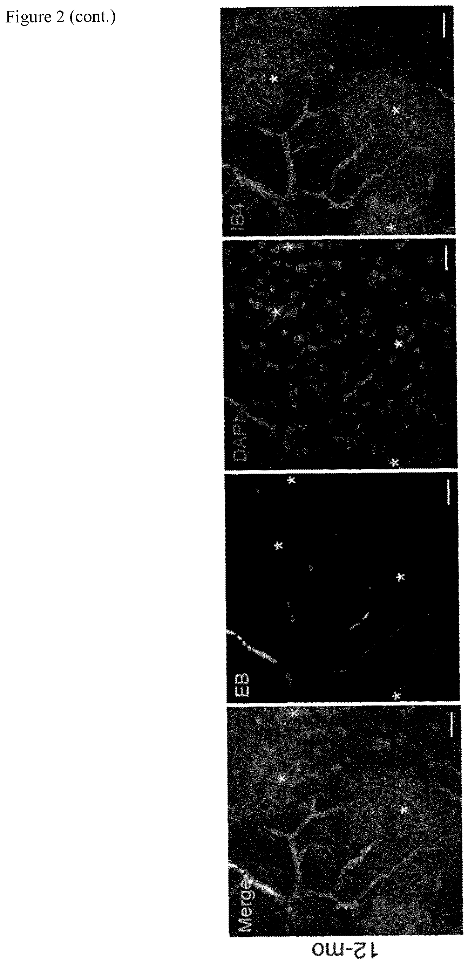

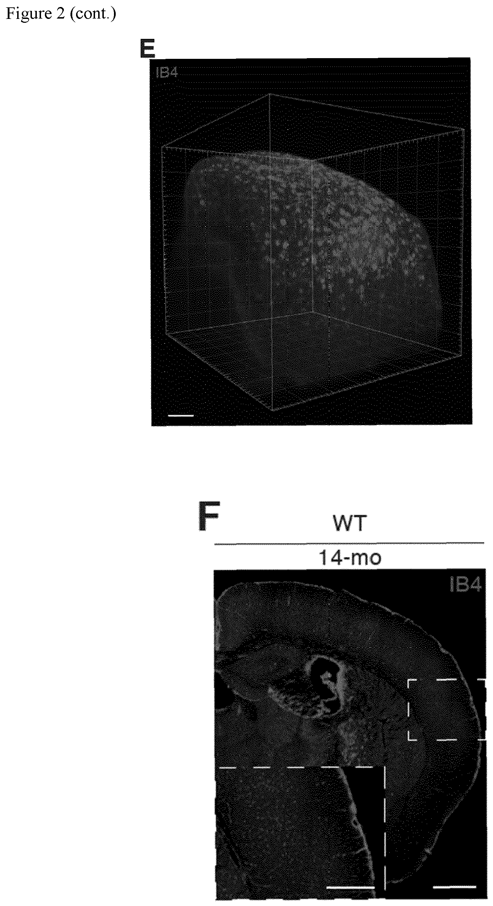

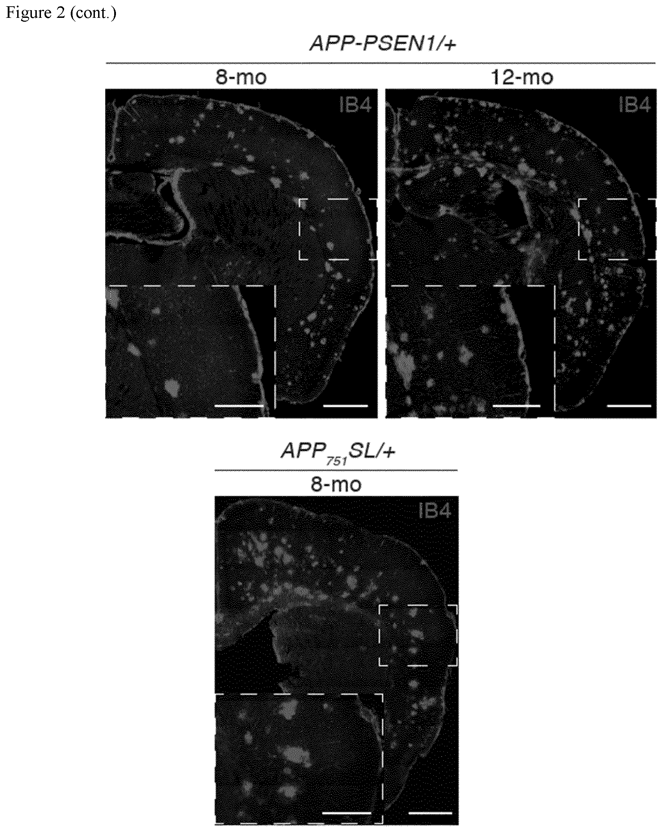

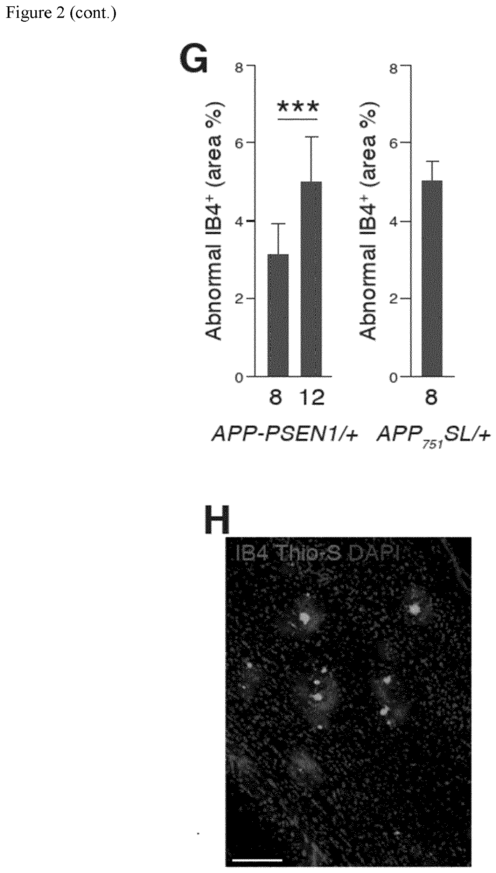

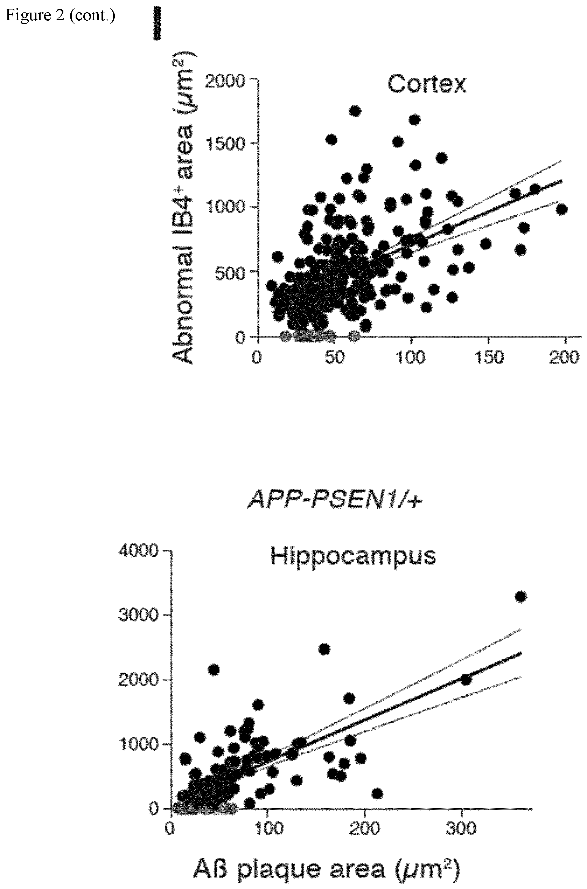

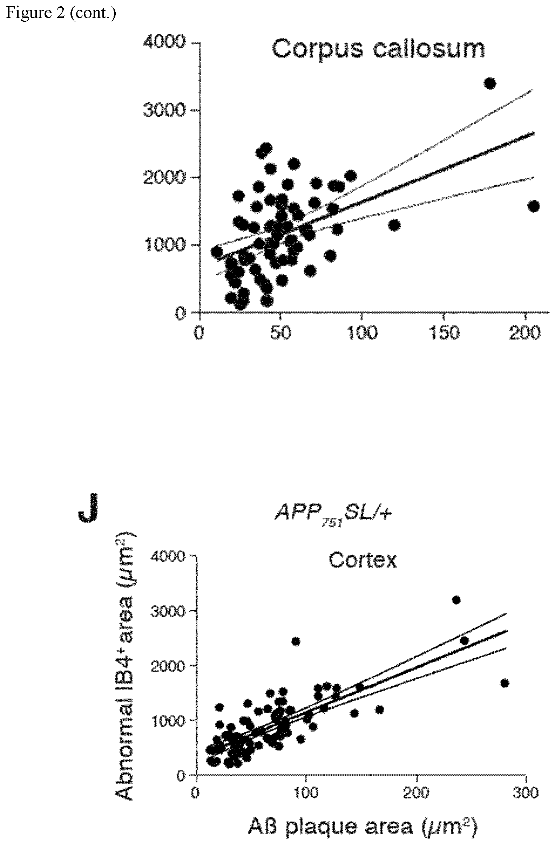

**Figure 2****. Angiogenesis is non-productive around Aβ plaques. (A)** Working model of the angiogenesis around Aβ plaques. Numeration continues from Fig. 1A. Non-productive angiogenesis will convert phalanx cells to non-conducting tip cells, extending local hypoxia (5). **(B, C)** Gene set enrichment analysis (GSEA) revealed that Dll4+/-_Up GS is highly represented in 18-month-old *APP-PSEN1*/+ versus wild-type (WT) endothelial cell differential transcriptomic (**B**, left panel). Right panel **(B)** shows the heat map of the top 30 ranking leading edge genes included in the Dll4+/-_Up GS. Red symbolizes overexpression and blue down regulation. **(C)** The table includes the GS enriched with an FDR *q*-value less than 0.05. **(D)** Confocal projection of coronal brain slices from 8- (upper row) and 12- (lower row) month-old (mo) *APP-PSEN1*/+ mice injected with Evans Blue (EB, white) and stained with endothelial (IB4; red) and nuclear (DAPI; blue) markers. Aβ plaques are autofluorescent in blue (yellow asterisks). Scale bars are 20 µm. **(E)** Full hemi-cortex from a 10-month-old *APP-PSEN1*/+ mouse stained to visualize endothelial cells (IB4; red) and rendered transparent using iDISCO. Scale bar is 500 µm. **(F)** Superimages of coronal brain sections from wild-type (WT), *APP-PSEN1*/+, and *APP₇₅₁SL*/+ mice stained to label endothelial cells (IB4; red). Insets show the white square from low magnification images. Scale bars are 1 mm in low and 500 µm in high magnification images. **(G)** Quantification of the percentage of cortical surface occupied by abnormal IB4⁺ staining. Mean ± SEM. *n* = 35 (8-mo; *APP-PSEN1*/+), 16 (12-mo; *APP-PSEN1*/+), and 18 (*APP₇₅₁SL*/+) cortical sections from respectively 3, 3, and 6 mice per experimental group; Student's *t*-test *** *p* < 0.001. **(H)** Image of a coronal brain slice from 8-mo *APP-PSEN1*/+ mice stained with endothelial (IB4; red), Aβ (Thioflavin-S; Thio-S; green), and nuclear (DAPI; blue) markers. Scale bar is 100 µm. **(I, J)** Fit of Aβ (Thio-S) plaque *versus* abnormal IB4⁺ areas from 8- and 12-mo (**I,** *APP-PSEN1*/+) and 8-mo (**J,** *APP₇₅₁SL*/+) coronal brain sections from stained as in **(H).** Thick lines represent the fit and the thin lines the 95% confidence interval. Red points indicate plaques without associated abnormal IB4⁺ areas. *APP-PSEN1*/+: Cortex: *n* = 263 plaques from 5 different mice. *r²* = 0.2487; *y* = 5.193x + 197.3; Spearman *r* correlation; *p* < 0.0001. Hippocampus: *n* = 125 plaques from 5 different mice. *r²* = 0.4672; *y* = 6.411x + 89.35; Spearman *r* correlation; *p <* 0.0001. Corpus callosum: *n* = 69 plaques from 5 different mice. *r²* = 0.2415; *y* = 9.7x + 660.7; Spearman *r* correlation; *p <* 0.0001. *APP₇₅₁SL*/+: Cortex: *n* = 90 plaques from 6 different mice. *r²* = 0.5943; *y* = 4.107x + 360.1; Spearman *r* correlation; *p* < 0.0001.

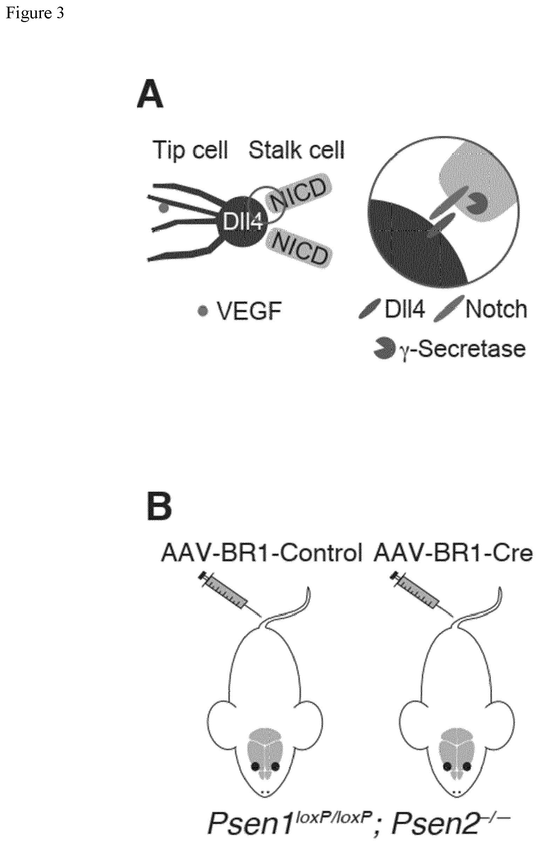

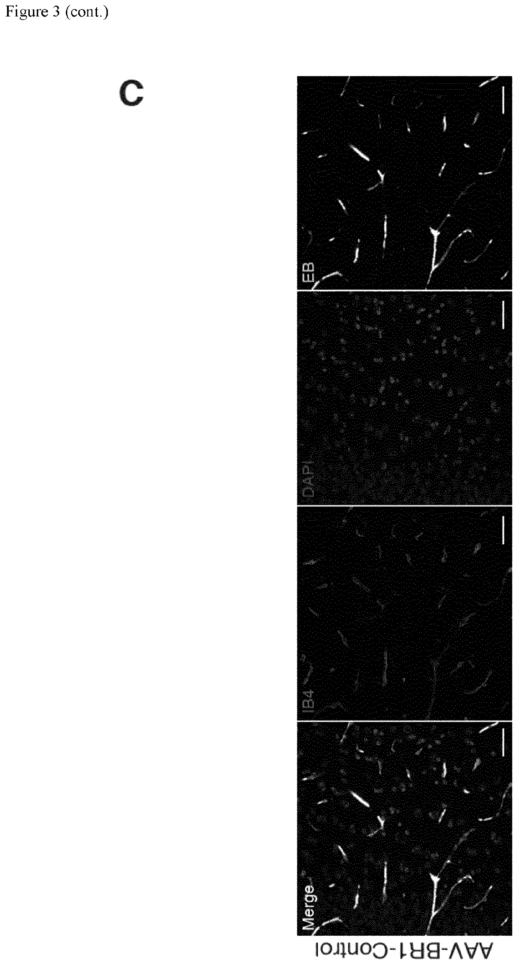

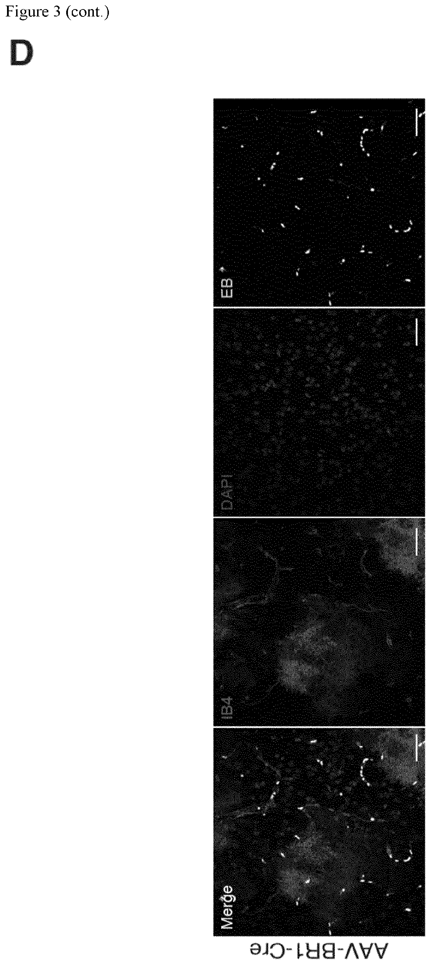

**Figure 3****. In adult inhibition of endothelial γ-secretase activity is enough to generate vascular anomalies. (A)** γ-secretase activity is involved in the lateral inhibition process that controls tip-stalk cells identity. **(B)** Schematic representation of a mouse model with adult inhibition of endothelial γ-secretase activity. *Psen1*^{*loxP*/*loxP*}; *Psen2*^{*-*/*-*} mice were injected with cerebral endothelium-specific adeno-associated control (AAV-BR1-Control) or Cre recombinase-expressing (AAV-BR1-Cre) viruses. **(C, D)** Confocal projections of coronal brain slices from 5-month-old *Psen1*^{*loxP*/*loxP*}; *Psen2*^{*-*/*-*} mice injected with AAV-BR1-Control **(C)** or AAV-BR1-Cre **(D),** and, two months later, perfused with Evans blue (EB; white) and stained with endothelial (IB4; red), and nuclear (DAPI; blue) markers. Scale bars are 40 µm.

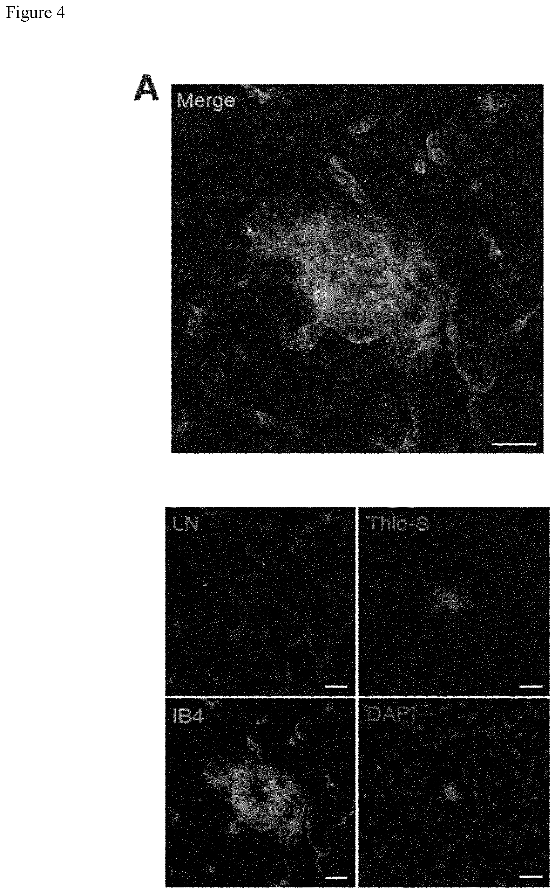

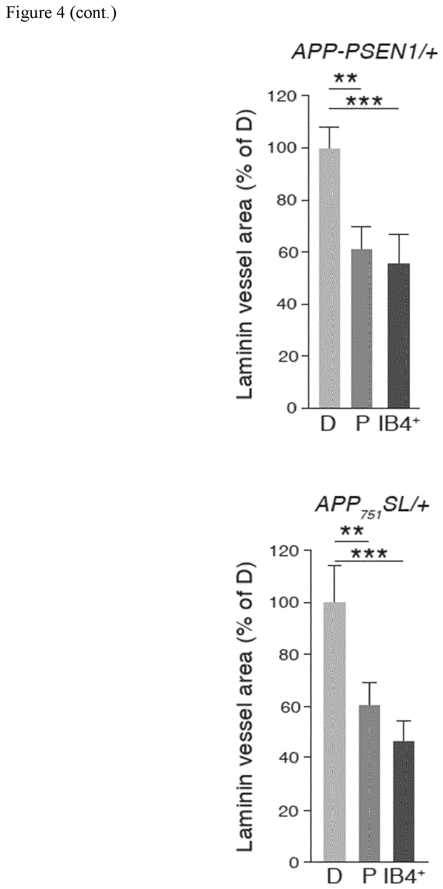

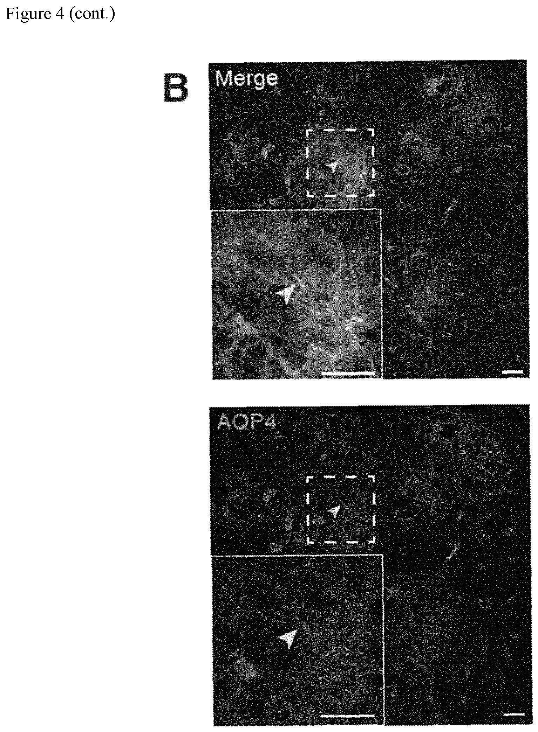

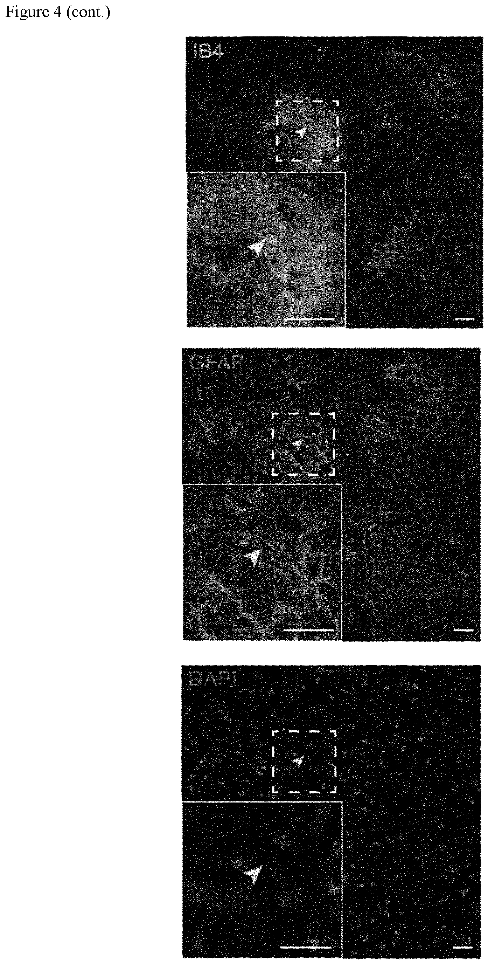

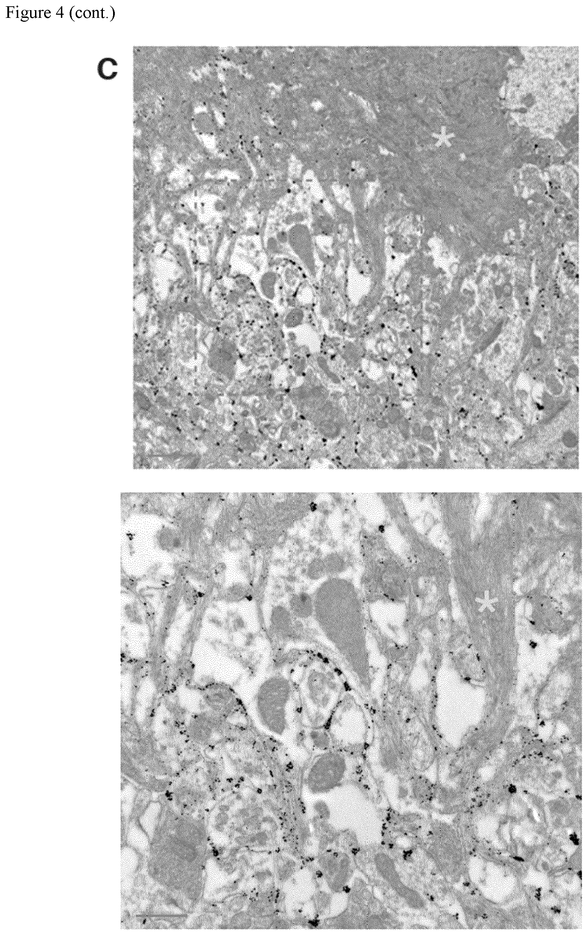

**Figure 4****. Vessels are substituted by vascular scars proximal to Aβ plaques. (A)** Left panels, confocal XY images of coronal brain slices from 8-month-old *APP-PSEN1*/+ mice stained with vessel basement membrane (laminin, LN; red), endothelial (IB4; white), Aβ (Thioflavin-S, Thio-S; green), and nuclear (DAPI; blue) markers. Aβ plaques are autofluorescent in blue. Scale bars are 20 µm. Right bar graphs, quantification of the laminin vessel area proximal (P) to Aβ plaques and inside the anomalous IB4⁺ structures (percentage of distal -D- laminin area) in 8-month-old *APP-PSEN1*/+ and *APP₇₅₁SL*/+ mice. Mean ± SEM. *n* = 20 (*APP-PSEN1*/+) and 25 (*APP₇₅₁SL*/+) paired D-P images from respectively 4 and 5 different mice; paired Student's *t*-test ** *p* < 0.01. **(B)** Confocal projection of coronal brain slices from 8-mo *APP-PSEN1*/+ mice stained with astrocytic end-feet (aquaporin 4, AQP4; green), endothelial (IB4; cyan), astrocytic (GFAP, red), and nuclear (DAPI; blue) markers. Aβ plaques are autofluorescent in blue. Insets show the white square from low magnification images. Yellow arrowheads indicate an astrocytic end-feet juxtaposed to an IB4⁺ structure. Scale bars are 20 µm. **(C)** Electron microscopy analysis of an 8-month-old *APP₇₅₁SL*/+ brain stained with IB4 (black dots, gold particles). Right image is a high magnification of the left dashed square shown in the left panel. A yellow asterisk indicates an Aβ plaque. Scale bar is 1 µm in low and 0.5 µm in high magnification images.

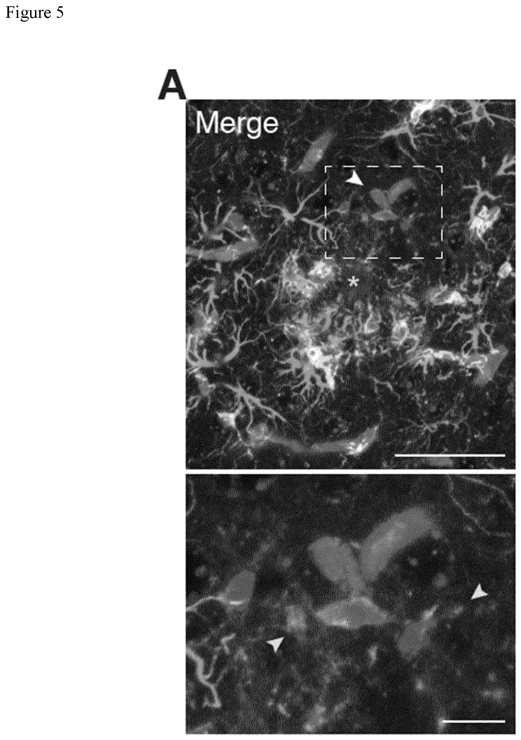

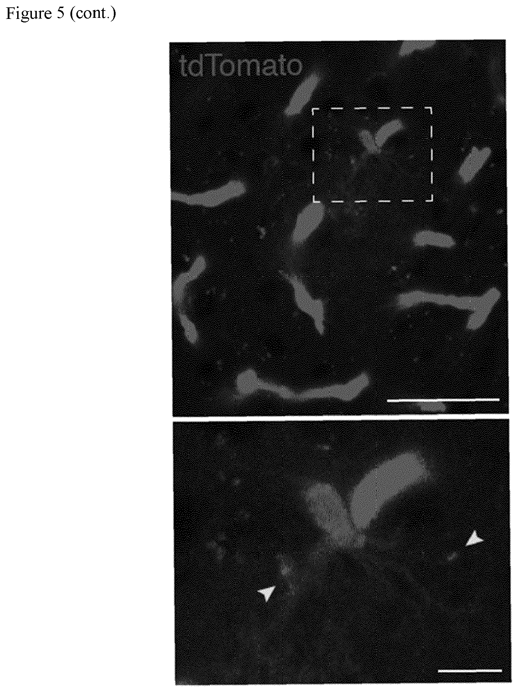

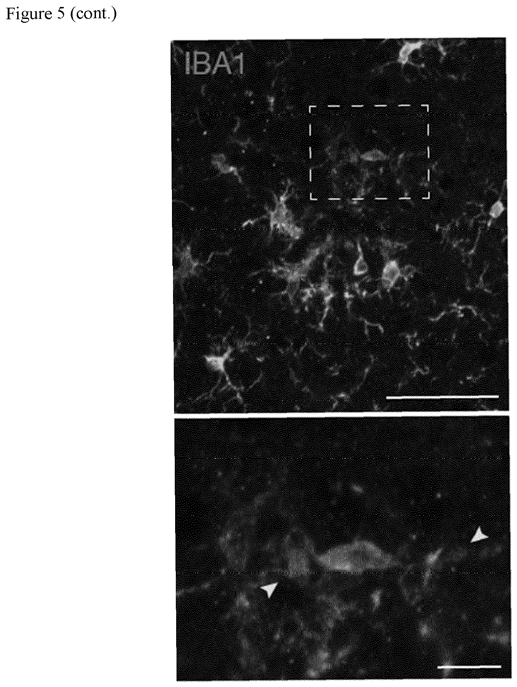

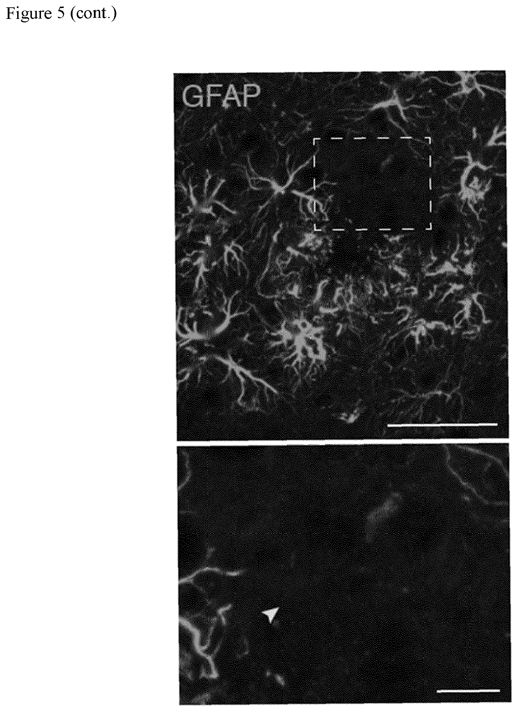

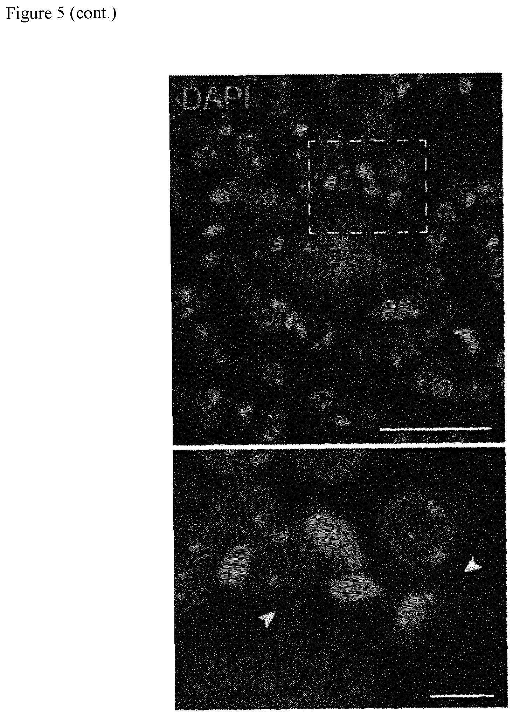

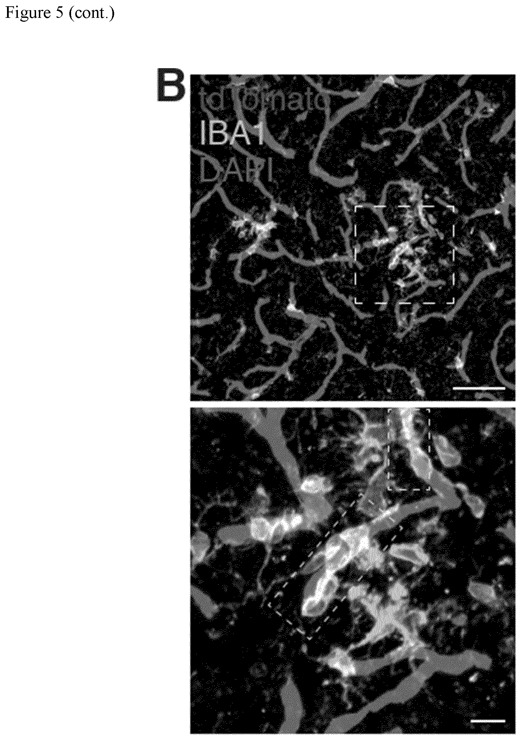

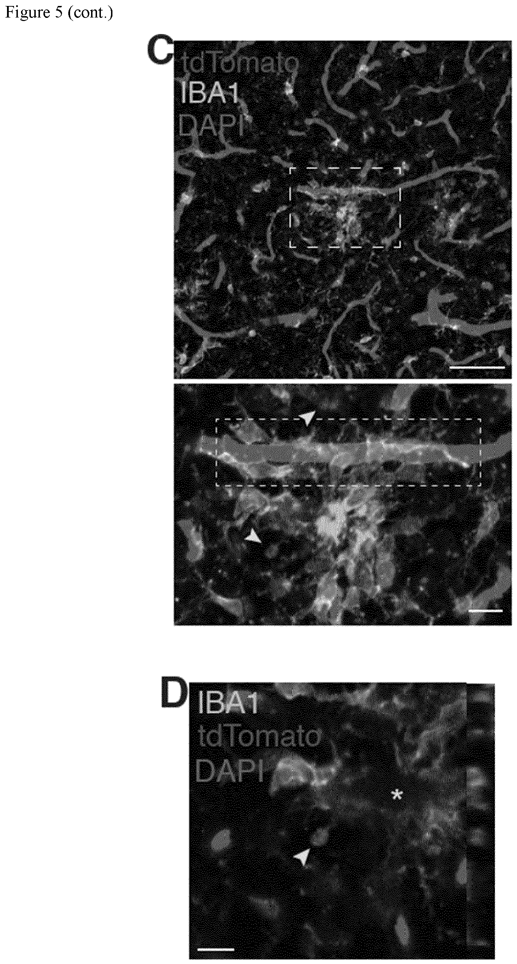

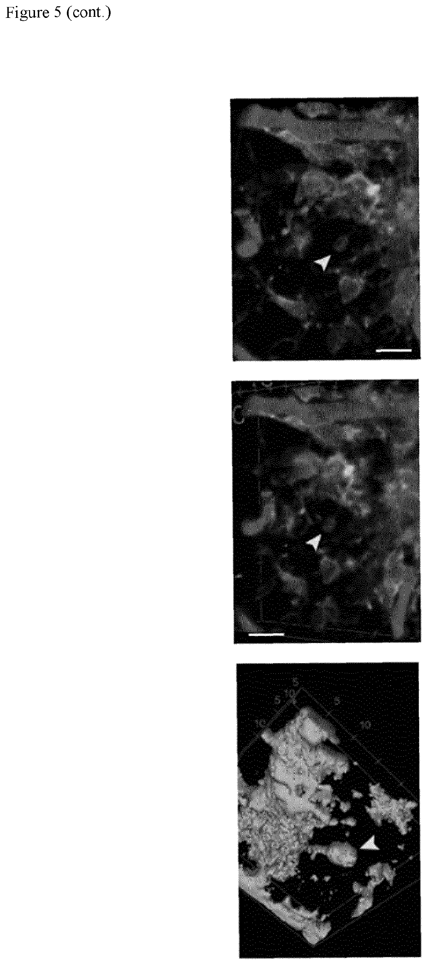

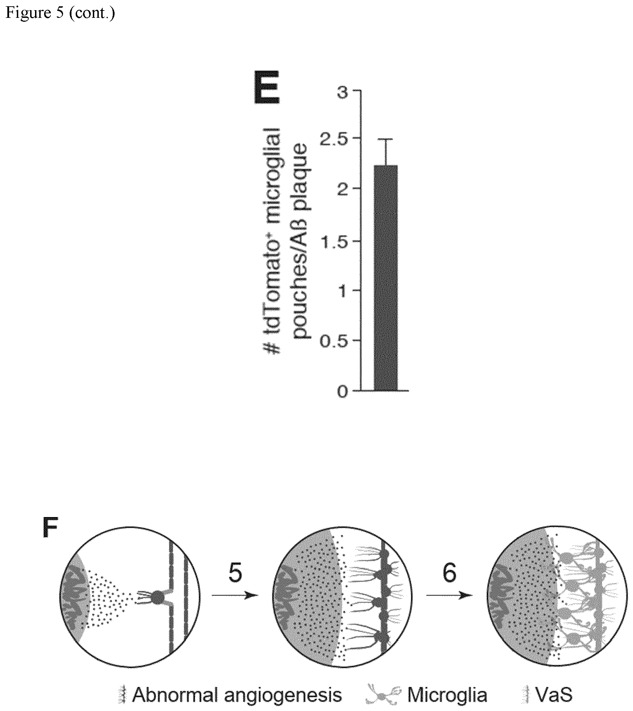

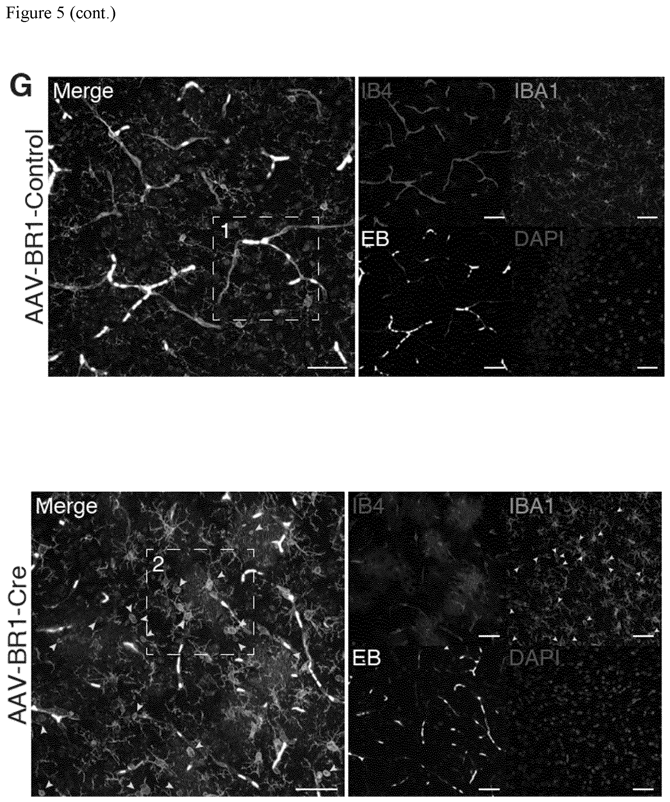

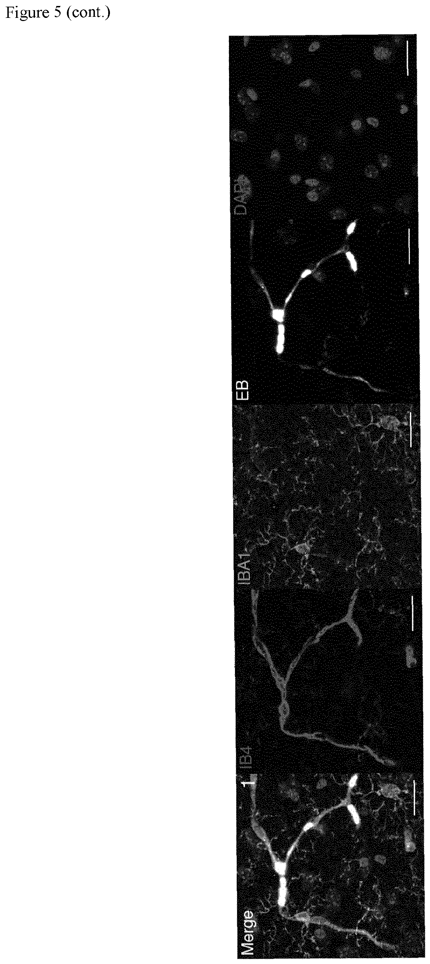

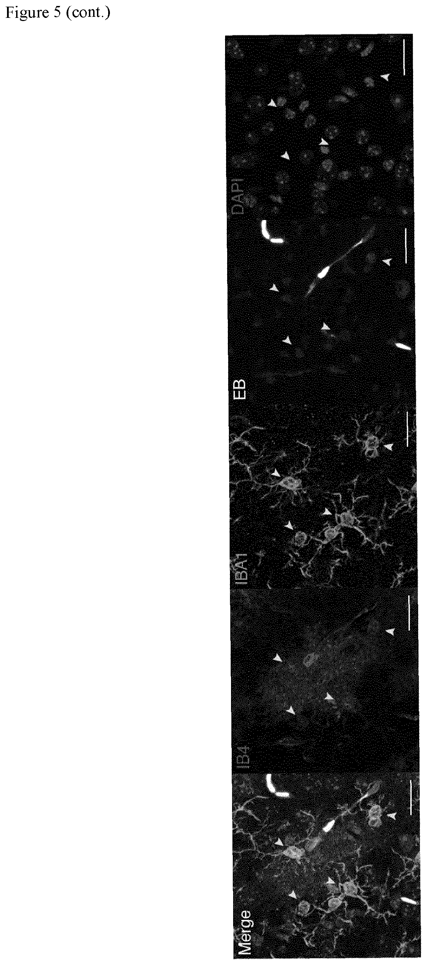

**Figure 5****. Microglial cells phagocyte blood vessels proximal to Aβ plaques. (A-D)** Confocal images of coronal brain slices from 8-month-old *Cdh5-Cre::ERT2*/*+; R26-LSL-tdTomato*/+; *APP-PSEN1*/+ tamoxifen-treated mice and stained with a tdTomato antibody **(A)** or direct tdTomato fluorescence **(B-D)** (red) and with microglial (IBA1; cyan), astrocytic (GFAP; green), and nuclear (DAPI; blue) markers. Aβ plaques are autofluorescent in blue (yellow asterisk). Yellow arrowheads indicate internalization of tdTomato⁺ signal by microglia (pouches). (A) Lower row images show the dashed white rectangles depicted in the upper row images. White arrowhead indicates a tip cell that projects extensions towards an Aβ plaque. Scale bars are 50 µm in low and 10 µm in high magnification images. **(B, C)** Lower row image shows the dashed white rectangles depicted in the upper row image. Regions with high alignment of endothelial cells and microglia are highlighted with dashed yellow rectangles. **(D)** left panel shows a z-projection of a magnified and cropped image from **(C)** showing the orthogonal projections. The right panels show different rotated views of 3D reconstructions of the left image (volume, two central panels; surface, right panel). Scale bars are 50 µm in low and 10 µm in high magnification images. **(E)** Quantification of the number of microglial pouches proximal to Aβ plaques. Mean ± SEM. *n* = 24. **(F)** Working model of the process of vascular scars (VaS) formation. Numeration continues from Fig. 1A. **(G)** Adult inhibition of endothelial γ-secretase activity (Fig. 3B) induces endothelial phagocytosis by microglia. Confocal projections of coronal brain slices from 5-month-old *Psen1*^{*loxP*/*loxP*}; *Psen2*^{*-*/*-*} mice injected with AAV-BR1-Control (left) or AAV-BR1-Cre (right), and, two months later, perfused with Evans blue (EB; white) and stained with endothelial (IB4; red), microglial (IBA1; green), and nuclear (DAPI; blue) markers. Yellow arrowheads indicate microglial pouches that contain IB4⁺ material. Lower rows are high magnification of the dashed white rectangles depicted in the upper row. Scale bars are respectively 40 µm and 20 µm in low and high magnification images.

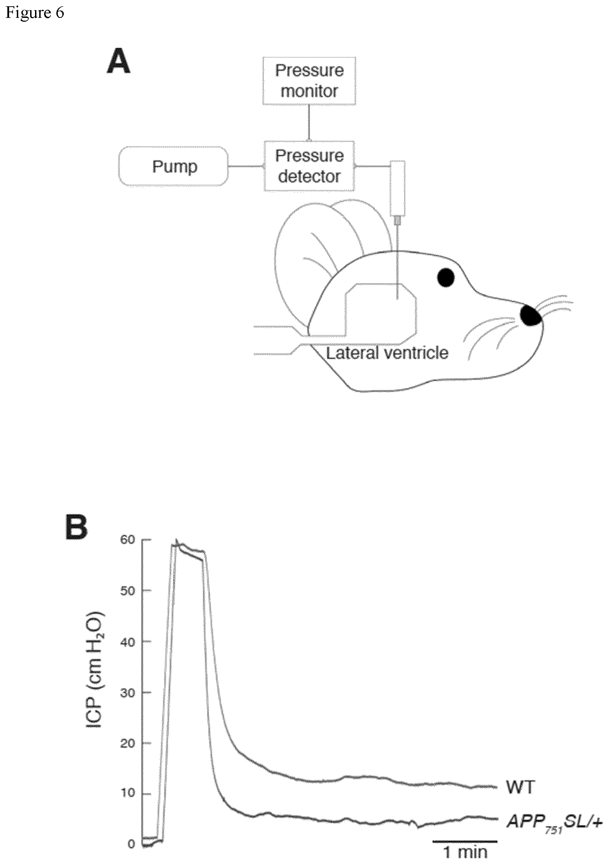

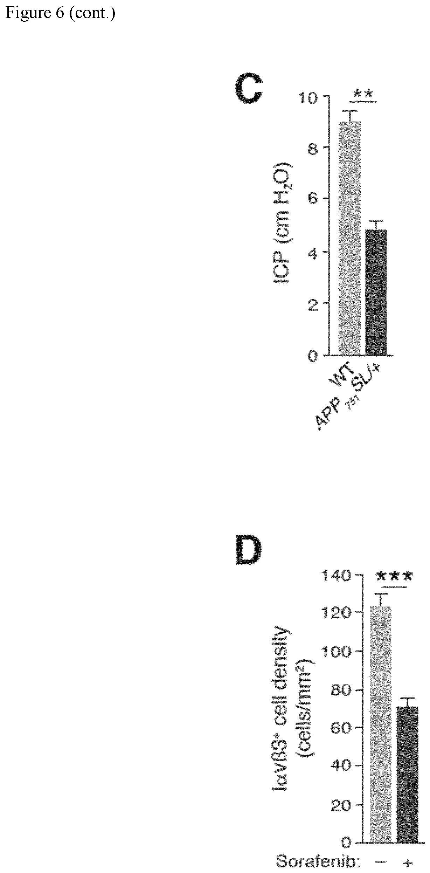

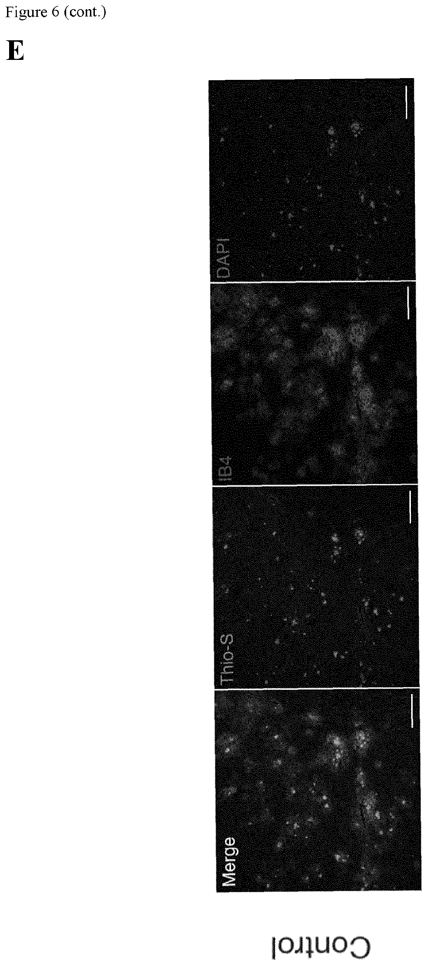

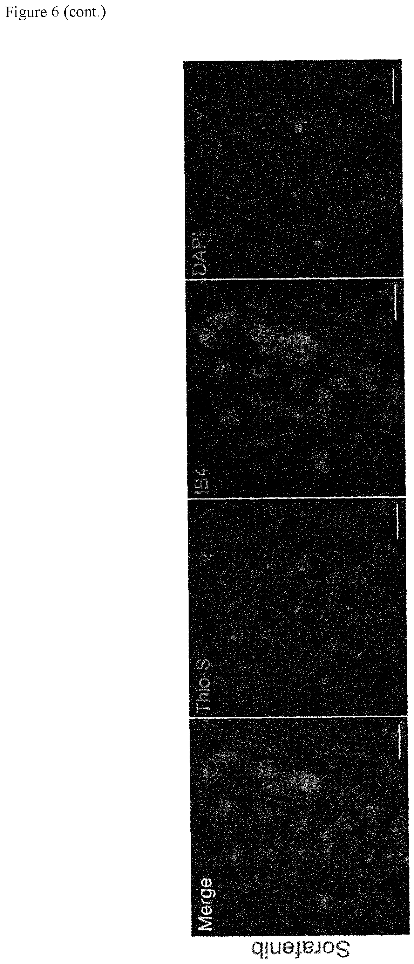

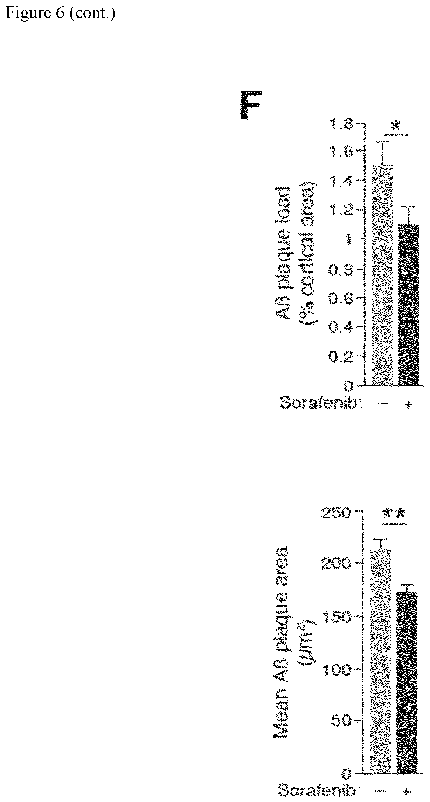

**Figure 6****. Inhibition of pathological angiogenesis improves Aβ clearance. (A-C)** Diminished intracerebral pressure (ICP) in *APP₇₅₁SL*/+ AD mice. **(A)** Diagram illustrating the procedure to measure the ICP. **(B)** Representative records of ICP from a wild-type (WT; blue line) and *APP₇₅₁SL*/+ (red line) mice. **(C)** Values of ICP in WT (blue bar) and *APP₇₅₁SL*/+ (red bar) mice. Mean ± S.E.M. *n* = 5 mice per genotype; Student's *t*-test ** *p <* 0.01. **(D, F)** 7-month-old *APP₇₅₁SL*/+ mice were treated with vehicle (-) or Sorafenib (+) for one month. *n =* 5 (-) and 6 (+) mice **(D)** Quantification of the Iαvβ3⁺ cell density in C (gray bar) and S (blue bar) -treated mice. *n =* 50 (-) or 60 (+) high power fields; Student's *t*-test *** *p <* 0.001. **(E)** Coronal brain slices stained with Aβ (Thioflavin-S, Thio-S; green), endothelial/VaS (IB4; white), and nuclear (DAPI; blue) markers. Aβ plaques are autofluorescent in blue. Scale bars are 200 µm. **(F)** Quantification of the Aβ plaque load (upper bar graph) and mean Aβ plaque area in (-) (gray bar) and (+) (blue bar) -treated mice. Mean ± S.E.M. *n* = 15 (C, 5 mice) or 16 (S, 6 mice) superimages; Student's *t*-test * *p* < 0.05; *** p* < 0.01.

### Detailed description of the invention

A detailed description of the invention is herein provided by means of the following examples, without the intention of limiting its scope of protection.

### Example 1. Angiogenesis is initiated around Aβ plaques in the AD brain.

To characterize the presence of pathologic angiogenesis around Aβ plaques we first investigated if these deposits induced the initiation of angiogenesis. The growing of new central nervous system vessels is normally instructed by the expression of VEGF in astrocytes **(****Figure 1A****),** several reports have shown that VEGF is upregulated in the human AD brain, and that VEGF protein localizes within Aβ plaques of AD mice. To distinguish the identity of the cells producing VEGF around Aβ we combined *in situ* hybridization with immunofluorescence for either the astrocytic marker glial fibrillary acidic protein (GFAP) or the ionized calcium binding adaptor molecule 1 (IBA1) microglial marker, another cell commonly found associated with Aβ plaques. Astrocytes were more frequently found expressing *Vegfa* mRNA around plaques than microglia, another cell commonly found associated with Aβ plaques **(****Figure 1B-****D)** and this VEGF production also correlated with the protrusion of filopodia from nearby vessels, suggesting angiogenic activity.

Next, we studied the expression of the integrin αvβ3 (Iαvβ3), a marker of angiogenic cells required for the stabilization of VEGFR2 upon binding of VEGF. A previous report described that Iαvβ3⁺ cells accumulated in the human AD brain and we verified that angiogenic cells were also abundant in the brain of *APP-PSEN1*/+ mice **(Figure IE).** In the human AD brain, Iαvβ3 was also highly expressed **(****Figure 1F****)** and combination of Thioflavin-S (Thio-S) staining with Iαvβ3 immunodetection suggested a connection between Iαvβ3⁺ cells and Aβ plaques **(****Figure 1F****).** To confirm the association between angiogenesis and Aβ deposits, we first localized the position of all the Iαvβ3⁺ cells and Aβ plaques (573 Aβ plaques and 2,995 Iαvβ3⁺ cells from 4 AD cases) and measured the load of the neurofibrillary tangles, another AD hallmark, in each AD case studied. An almost perfect direct correlation was observed between the number of Iαvβ3⁺ cells and Aβ plaques and, on the contrary, no association was found between Iαvβ3⁺ cells and tangles. Second, we performed *in silico* simulations to determine if both events, Iαvβ3⁺ cells and Aβ plaques, were spatially associated. In each section, we performed 500 simulation where the position of Aβ plaques was conserved and the location of the Iαvβ3⁺ cells was randomly generated. We then measured the shortest geodesic distance between each Iαvβ3⁺ cell and the closest Aβ plaque in each random simulation and in the experimental cases. Notably, in the four cases studies, the shortest distance between Iαvβ3⁺ cells and Aβ plaques was always significantly smaller in the experimental case than in the random simulations and, globally, the shortest distance between Iαvβ3⁺ cells and Aβ plaques was also significantly smaller to that expected in a random distribution of Iαvβ3⁺ cells **(****Fig. 1G****).** Altogether, the local induction of VEGF expression by astrocytes and the concentration of angiogenic cells nearby Aβ plaques strongly suggest that angiogenesis is initiated around Aβ deposits in the human AD brain and in AD mouse models.

### Example 2. Non-productive angiogenesis (NPA) around Aβ plaques.

As described before, a puzzling characteristic of the AD brain is the accumulation of angiogenic markers coupled with a reduction in the number of vessel and a debilitation of the BBB. To investigate if angiogenesis is halted by a failure in differentiation (NPA) in AD mouse models **(****Figure 2A****),** we developed a protocol to isolate endothelial cells from aged (18-month-old) *APP-PSEN1*/+ and wild-type mice. Endothelial cells are developmentally derived from an angioblast, which in turn derives from a hemangioblast, a mesoderm-derived progenitor cell that also gives rise to the hematopoietic stem cells. We separated CD31 positive (⁺) (an endothelial/innate immune cell marker) and CD11b negative (⁻) (an innate immune marker) cells using fluorescence-activated cell sorting (FACS). mRNA levels of a vascular specific marker (*cadherin* 5, *Cdh5*) greatly exceeded the levels of microglia, astrocyte, oligodendrocyte, and neuronal markers, confirming the purity of the isolated endothelial cells. A global expression analysis was performed with these isolated endothelial cells. To evaluate whether endothelial cells from an Aβ-accumulating AD mouse model could suffer NPA (reduction of Notch-mediated lateral inhibition), we defined a gene set (GS) containing the genes upregulated in the retina of *Dll4* heterozygous mice (Dll4+/-_Up) and estimated its contribution to the DE genes between *APP-PSEN1*/+ and wild-type endothelial cells using GS enrichment analysis (GSEA). Notably, the Dll4+/-_Up GS was enriched at the top of the list - and the single one with a significant FWER-*p*-value- out of more than 800 GS analysed **(****Figure 2B, C****),** strongly suggesting that angiogenic tip cells fail to differentiate neighbour endothelial cells into stalk cells, the cell type that should proliferate, generate the lumen of the new vessel, and stablish the proper BBB.

In the search of vascular alterations associated with the halted angiogenesis predicted by the molecular signature, we used the isolectin B4 (IB4) from Griffonia simplicifolia, a well-described marker of mouse mature and angiogenic endothelial cells, and of the vascular abnormalities observed in NPA models. As expected, IB4 delineated the vessels distal to Aβ plaques **(****Figure 2D****),** however, around Aβ deposits IB4 recognized an abnormal structure with reduced perfusion (angiography with Evans blue), a "cotton candy"-like appearance, and that emanated from well-perfused IB4⁺ vessels **(****Figure 2D****).** The anomalous IB4⁺ structures were found all along the cortex, hippocampus, and the corpus callosum of the *APP-PSEN1*/+ and *APP₇₅₁SL*/+ mice **(****Figure 2E-F****)** and quantification of the brain area occupied by the abnormal IB4⁺ staining revealed that the cortical coverage increased up to 5% of the total surface from 8- to 12-month-old *APP-PSEN1*/+ mice **(****Figure 2G****).** In the brain of *APP₇₅₁SL*/+, a mouse model with a faster rate of Aβ deposition, a similar IB4⁺ cortical load was already observed in 8-month-old mice **(****Figure 1G****),** suggesting an association between the anomalous structures and Aβ plaques. To quantitatively confirm the connection between the abnormal IB4⁺ structures and Aβ plaques, we co-stained brain slices from Aβ-depositing AD mouse models with thioflavin-S (Thio-S) and IB4⁺. The anomalous IB4⁺ staining was localized surrounding Aβ plaques **(****Figure 2H****)** and quantification of the area of both Aβ deposits (Thio-S+) and the atypical IB4⁺ structures revealed a significant positive correlation between both parameters in the three regions analysed (**Figure 2I****;** cortex, hippocampus and corpus callosum of 8- and 12-month-old *APP-PSEN1*/+ mice), which was also confirmed in the cortex of the *APP₇₅₁SL*/+ mice **(****Figure 2J****).** In only a few examples, Aβ plaques were devoid of abnormal vascular structures (10 out of 263 plaques in cortex, 15 out of 125 in hippocampus, and 0 out of 69 in corpus callosum). However, the number of IB4⁺ structures non-associated with Aβ plaques was negligible and could possibly be accounted for a plaque above or below the sectioning plane where the IB4 staining was found (sectioning thickness was 40 µm).

The morphological consequences of NPA has so far been characterized during the development of the mouse brain, in the retina of perinatal mice, or in tumors. To investigate if in adult inhibition of the Notch signalling pathway **(****Figure 3A****)** in brain endothelial cells could produce vascular alterations similar to the Aβ plaque-associated abnormal IB4⁺ structures in the absence of Aβ pathology, we genetically inhibited endothelial γ-secretase activity by injecting *Psen1*^{*loxP*/*loxP*}; *Psen2*^{*-*/*-*} mice with cerebral endothelium-specific control or Cre recombinase-expressing adeno-associated viruses (AAV-BR1; **Figure 3B**). As expected, a normal brain microvasculature was observed in mice injected with the control vector **(****Figure 3C****),** however, the morphology of the vessels was deeply altered in the brain of mice injected with Cre-expressing AAVs (AAV-BR1-Cre; **Figure 3D**). Notably, the abnormal IB4⁺ structures observed were identical to that observed around Aβ plaques (compare **Figure 2D-G** with **Figure 3D**), even though the mouse model employed did not overexpressed APP and therefore did not accumulate Aβ deposits. Altogether, our results strongly support that Aβ plaques induce NPA in endothelial cells, which generates anomalous IB4⁺ vascular structures.

### Example 3. NPA induces vessel loss and accumulation of vascular scars (VaS).

To study if Aβ plaques-associated NPA could explain the reduced number of capillaries and the alteration of the BBB described in the human AD brain and in AD mouse models, we used laminin -a marker of the endothelial basement membrane- in combination with IB4. As expected, both laminin and IB4 delineated the vessels distal to Aβ plaques **(****Figure 4A****),** however, laminin staining was reduced in the anomalous IB4⁺ structure. We quantified the area occupied by laminin⁺ vessels in a 50 µm radius from Aβ plaques and observed a significant reduction in two different mouse models of Aβ deposition **(****Figure 4B****).** Interestingly, the decrease in laminin⁺ area was similar when quantified inside the abnormal IB4⁺ area **(****Figure 4B****),** suggesting that blood vessels were disassembled into those atypical structures in the immediate proximity to Aβ plaques. To further characterize the relation between vessel loss and the anomalous IB4⁺ structures, we use several additional vascular unit markers. Similar to laminin, the platelet/endothelial cell adhesion molecule 1 (PECAM1/CD31; a tight junction marker) decreased its expression in the proximity to Aβ deposits in the area occupied by the abnormal IB4⁺ structures but, as expected, colocalized with the IB4 staining in regions distal to Aβ plaques. Pericyte number is decreased in both patients and AD models and the reduction correlates with Aβ deposits in AD brains. Correspondingly, platelet derived growth factor receptor β (PDGFRβ, a pericyte marker) expression was observed in vessels distal to Aβ plaques and loss in the anomalous IB4⁺ structures. Finally, Aquaporin 4 (AQP4), a marker of astrocytic end-feet, delineated the blood vessels in regions distal to Aβ plaques **(****Figure 4B****),** but a diffuse signal was observed in the proximity of Aβ deposits that colocalized with the anomalous IB4⁺ structures in some areas **(****Figure 4B****),** including clearly recognizable-astrocytic end-feet (arrowheads in **Figure 4B**). Based on their continuity from perfused blood vessels, their association with the loss of mature vessel markers, and their diffuse accumulation around the Aβ deposits, we termed these abnormal IB4⁺ structures Vascular Scars (VaS). To map IB4 staining at the ultrastructural level, we used gold-based IB4 staining and electron microscopy. IB4 labelling was mainly restricted to the extracellular space surrounding Aβ fibrils **(****Figure 4C****),** a parenchyma that is normally invaded by microglial and astrocytic projections.

But, how halted angiogenesis induced vessel loss? To answer this question, we generated a new AD mouse model where tdTomato was conditionally expressed in the cytoplasm of endothelial cells (*APP-PSEN1*/+; *Cdh5-Cre::ERT2*/+; *tdTomato*/+) upon tamoxifen (TMX) treatment. Non-treated mice did not show any tdTomato staining (data not shown) and blood vessels were clearly identified in brain sections of TMX-treated wild-type and *APP-PSEN1*/+ mice. In addition, we also observed, although with low frequency, tdTomato⁺ cells extruding from blood vessels **(****Figure 5A****),** which could represent non-terminally differentiated tip/stalk cells. Interestingly, those cells were found in close apposition to microglia **(****Figure 5A****)** and their projections were total or partially covered by microglial cytoplasmic extensions **(****Figure 5A****).** To confirm that microglia are indeed involved in the local loss of Aβ plaque-associated blood vessels, we first studied the spatial distribution of microglial and endothelial cells in the absence of pathology (wild-type mice) and distal and proximal to Aβ plaques in an AD mouse model. Low magnification images revealed that Aβ plaque-associated microglia (AβAM) were found covering blood vessels **(****Figure 5B****,** **C****),** something that was not observed in wild-type mice or in microglia distal to Aβ deposits. Interestingly, tdTomato⁺ microglial pouches -ball- and-chain structures characteristic of phagocytic microglia- were clearly identified proximal to Aβ deposits **(****Figure 5C, D****)** with a high frequency **(****Figure 5E****).** Altogether, our data suggest that induction of NPA induces microglial phagocytosis and local vessel loss **(****Figure 5F****).** Finally, to investigate whether reduced lateral inhibition was sufficient to initiate AβAM-mediated phagocytosis of blood vessels, we went back to our NPA model in the absence of Aβ deposits (*Psen1*^{*loxP*/*loxP*}; *Psen2*^{*-*/*-*} mice injected with AAV-BR1-Cre). Interestingly, adult inhibition of endothelial γ-secretase activity induced a thickening of microglial projections **(****Figure 5G****)** and the appearing of circular cytoplasmic microglial pouches that enveloped IB4⁺ material **(****Figure 5G****).** Astrocytic projections were not involved in the remodelling of γ-secretase deficient blood vessels, although an increase in GFAP expression was observed all around the brain regions studied, suggesting the presence of local damage associated with VaS. Altogether, our data indicate that induction of NPA is sufficient to disassemble blood vessels and to induce endothelial cell phagocytosis by microglia.

### Example 4. NPA compromises Aβ plaques clearance.

Extracellular Aβ can be cleared from the brain by several systems, including the BBB, the interstitial fluid (ISF) bulk flow -involving the glymphatic system-, and the meningeal lymphatic vessels. The loss of the brain microvascular network and the disorganization of the AQP4 expression proximal to Aβ plaques **(****Figure 4B****)** suggest that the clearance of Aβ not only by the BBB but also by the ISF bulk flow can be compromised. Therefore, we analysed the intracranial pressure (ICP) as a subrogate indicator of the ISF-CSF relationship by stereotaxic puncture of the lateral ventricle with a microneedle in 8-month-old wild-type and *APP₇₅₁SL*/+ mice **(****Figure 6A****).** *APP₇₅₁SL*/+ mice showed a significant decrease in ICP **(****Figure 6B****)** without changes in CSF outflow and ventricular compliance, strongly suggesting that the vessel loss observed around Aβ plaques compromises ISF bulk flow.

### Example 5. NAP inhibitors improve the clearance of Aβ.

We have shown that angiogenesis is initiated albeit not completed in the brain of AD mouse models, leading to vessel loss around Aβ plaques. We believe that we could prevent the loss of blood vessels in newly deposited Aβ plaques by blunting the initiation of pathologic angiogenesis (NPA), which could improve the clearance of Aβ. To this end, we selected Sorafenib as a small drug that inhibits the intracellular activity of several angiogenic kinases (VEGFR, PDGFR, and RAF) in endothelial cells. Sorafenib treatment (1 month) strongly reduced the apparition of Iαvβ3⁺ cells in the brain of 7-month-old *APP₇₅₁SL*/+ mice **(****Figure. 6C****)** indicating that the antiangiogenic activity of the drug was reached with the selected treatment. The Aβ plaques in the brain were examined using Thio-S staining and it was revealed that Sorafenib significantly reduced the Aβ plaque load and the mean Aβ plaque area **(****Figure 6D****,** **E****),** strongly suggesting that blocking of the Aβ plaque-associated blood vessel disassemble by NPA improves Aβ clearance and alleviates the local pathology.

## Claims

1. Non-productive angiogenesis inhibitor for use in the treatment of Alzheimer's disease.

2. Non-productive angiogenesis inhibitor for use, according to claim 1, wherein the inhibitor is selected from the group consisting of: Sorafenib, Axitinib, Cabozantinib, Everolimus, Lenalidomide, Pazopanib, Regorafenib, Sunitinib, Thalidomide, Vandetanib, Ziv-aflibercept, Lapatinib, Lenvatinib, Ponatinib, Dasatinib, Nilotinib, Regorafenib or any combination thereof.

3. Sorafenib for use, according to any of the previous claims, in the treatment of Alzheimer's disease.

4. Non-productive angiogenesis inhibitor for use, according to any of the previous claims, wherein the differentiation of endothelial cells into tip cells is halted and the abnormal vasculature is normalized.

5. Pharmaceutical composition comprising non-productive angiogenesis inhibitors and, optionally, pharmaceutically acceptable excipients or carriers, for use in the treatment of Alzheimer's disease.

6. Pharmaceutical composition for use, according to claim 5, wherein the inhibitor is selected from the group consisting of: Sorafenib, Axitinib, Cabozantinib, Everolimus, Lenalidomide, Pazopanib, Regorafenib, Sunitinib, Thalidomide, Vandetanib, Ziv-aflibercept, Lapatinib, Lenvatinib, Ponatinib, Dasatinib, Nilotinib, Regorafenib or any combination thereof.

7. Pharmaceutical composition for use, according to any of the claims 5 or 6, comprising Sorafenib and, optionally, pharmaceutically acceptable excipients or carriers.

8. *In vitro* method for the identification and production of candidate compounds for the treatment of Alzheimer's disease comprising: a) determining whether the non-productive angiogenesis has been inhibited, or the abnormal vasculature has been normalized, after administering the candidate molecule, b) where, if after administering the candidate molecule, the non-productive angiogenesis is inhibited, or the abnormal vasculature has been normalized, this is indicative that the candidate molecule is effective in the treatment of Alzheimer's disease.

9. *In vitro* method for monitoring the response to a treatment against Alzheimer's disease comprising: a) determining whether the non-productive angiogenesis has been inhibited, or the abnormal vasculature has been normalized, after the administration of the treatment, b) where, if after administering the treatment, the non-productive angiogenesis is inhibited, or the abnormal vasculature has been normalized, this is indicative that the treatment is effective against Alzheimer's disease.

10. *In vitro* method, according to any of the claims 8 or 9, which comprises: a) determining whether the non-productive angiogenesis has been inhibited, or the abnormal vasculature has been normalized, after administering the candidate molecule, by measuring the number of filopodia emitting cells and/or the number of filopodia per cell, as indicative of the presence of tip cells, b) wherein, if after administering the candidate molecule, the number of tip cells is reduced, this is indicative that the candidate molecule is effective in the treatment of Alzheimer's disease or that the treatment is effective against Alzheimer's disease.

11. *In vitro* method, according to claim 10, wherein the presence or the number of tip cells are determined by means of IB4 staining and/or laminin expression.
